(19) **Europäisches Patentamt**
**European Patent Office**
**Office européen des brevets**

(11) **EP 2 868 287 B1**

(12) **EUROPEAN PATENT SPECIFICATION**

(45) Date of publication and mention
of the grant of the patent:
**03.05.2017 Bulletin 2017/18**

(51) Int Cl.:
*A61B 18/18* (2006.01)  *A61N 5/02* (2006.01)
*A61B 18/00* (2006.01)

(21) Application number: **14194675.6**

(22) Date of filing: **24.03.2010**

(54) **Apparatus for tissue sealing**

Vorrichtung zum Versiegeln von Gewebe

Appareil pour l'étanchéité de tissus

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **24.03.2009 US 410195**

(43) Date of publication of application:
**06.05.2015 Bulletin 2015/19**

(62) Document number(s) of the earlier application(s) in accordance with Art. 76 EPC:
**10157500.9 / 2 233 098**

(73) Proprietor: **Covidien LP**
**Mansfield, MA 02048 (US)**

(72) Inventors:
• **Nau, William H.**
**Longmont, CO 80504 (US)**
• **Rossetto, Francesca**
**Longmont, CO 80504 (US)**

(74) Representative: **Soames, Candida Jane et al**
**Maschio & Soames IP Limited**
**30 Carlton Crescent**
**Southampton SO15 2EW (GB)**

(56) References cited:
**WO-A1-2008/044000    JP-A- 2008 054 926**
**US-A1- 2007 225 697**

## Description

## BACKGROUND

*Technical Field*

The present disclosure relates to forceps for sealing various types of tissue. More particularly, the present disclosure relates to open, laparoscopic or endoscopic forceps that utilize microwave energy to seal tissue.

*Description of the Related Art*

In many surgical procedures, body vessels, e.g., blood vessels, ducts, adhesions, fallopian tubes, etc. are sealed to defunctionalize or close the vessel. Traditionally, staples, clips or sutures have been used to close a body vessel. However, these traditional procedures often leave foreign body material inside a patient. In an effort to reduce foreign body material left within the patient and to more effectively seal the body vessel, energy techniques that seal by heat processes have been employed.

A forceps is particularly useful for sealing tissue and vessels since forceps utilizes mechanical action to constrict, grasp, dissect and/or clamp tissue. Current vessel sealing procedures utilize heat treatment to heat and desiccate tissue causing closure and sealing of the body vessel. In addition, forceps allow for control of the applied pressure to the tissue. The combination of heating and applied pressure provides a uniform, controllable seal and that is capable of providing such a seal with minimum collateral damage to body tissue.

US2007/0225697 discloses an apparatus having adaptable clamps for forming encircling and linear lesions. To that end, a microwave antenna is disposed within a hollow chamber or recess within the jaw(s) of the clamp, along substantially the entire length of the jaw(s).

WO2008/044000 discloses a surgical cutting element comprising a blade and a plurality of patch antennas arranged in proximity of the cutting edge of the blade to emit a substantially uniform microwave radiation field at the edge.

JP2008 054926 discloses a microwave forceps in which a microwave antenna in the form of a wire is provided on the jaws' sealing surface. The wire is insulated except for the front end where micro waves should be emitted.

## SUMMARY

The present invention is defined in claim 1 with preferred embodiments as in the dependent claims.

The present disclosure provides for a microwave forceps for sealing tissue. The forceps includes a shaft member having an end effector assembly disposed at a distal end thereof. The end effector assembly includes opposing jaw members movable from a first position in spaced relation relative to one another to at least one subsequent position wherein the jaw members cooperate to grasp tissue therebetween. Each of the jaw members includes a sealing surface, wherein one of the sealing surfaces includes one or more microwave antenna assemblies coupled to a source of microwave energy.

The microwave antenna assembly may be coupled to a microwave energy source and may include a grounding member coupled to a ground reference of the microwave energy source and disposed within the first jaw member; a dielectric substrate disposed on the grounding member; and a patch antenna coupled to an active element of the microwave energy source and disposed on the dielectric substrate.

According to a further aspect of the present disclosure, the microwave antenna assembly may include: a slot antenna having a substantially rectangular slot defined therethrough, the rectangular slot having a first longitudinal side coupled to a ground reference of the microwave energy source and a second longitudinal side coupled to an active element of the microwave energy source.

According to another aspect of the present disclosure, a microwave forceps for sealing tissue is disclosed. The forceps includes a shaft member having an end effector assembly disposed at a distal end thereof. The end effector assembly includes opposing jaw members movable from a first position in spaced relation relative to one another to at least one subsequent position wherein the jaw members cooperate to grasp tissue therebetween. Each of the jaw members includes a sealing surface, wherein one of the sealing surfaces includes one or more microwave antenna assemblies coupled to a source of microwave energy. wherein the microwave antenna assembly is configured to operate in a therapeutic mode to deliver microwave energy to tissue and in a detection mode to measure at least one tissue property.

## BRIEF DESCRIPTION OF THE DRAWINGS

Various embodiments of the present disclosure are described herein with reference to the drawings wherein:

Fig. 1 is a perspective view of a tissue sealing system including a forceps and an energy generator according to one embodiment of the present disclosure;
Fig. 2 is a cross-sectional view of a distal end of the forceps of Fig. 1;
Figs. 3A-3B are views of a microwave end effector assembly according to one embodiment of the present disclosure;
Figs. 4A-4B are views of a microwave end effector assembly according to another embodiment of the present disclosure;

Figs. 5A-5B are views of a microwave end effector assembly according to another embodiment of the present disclosure; and

Figs. 6A-6C are views of a microwave end effector assembly according to another embodiment of the present disclosure.

## DETAILED DESCRIPTION

Various embodiments of the present disclosure are described hereinbelow with reference to the accompanying drawings. Well-known functions or constructions are not described in detail to avoid obscuring the present disclosure in unnecessary detail. Those skilled in the art will understand that the present disclosure may be adapted for use with either an endoscopic instrument or an open instrument; however, different electrical and mechanical connections and considerations apply to each particular type of instrument. The novel aspects with respect to vessel and tissue sealing are generally consistent with respect to both the open and endoscopic designs. In the drawings and in the description that follows, the term "proximal", as is traditional, will refer to the end of the forceps that is closer to the user, while the term "distal" will refer to the end of the forceps that is further from the user.

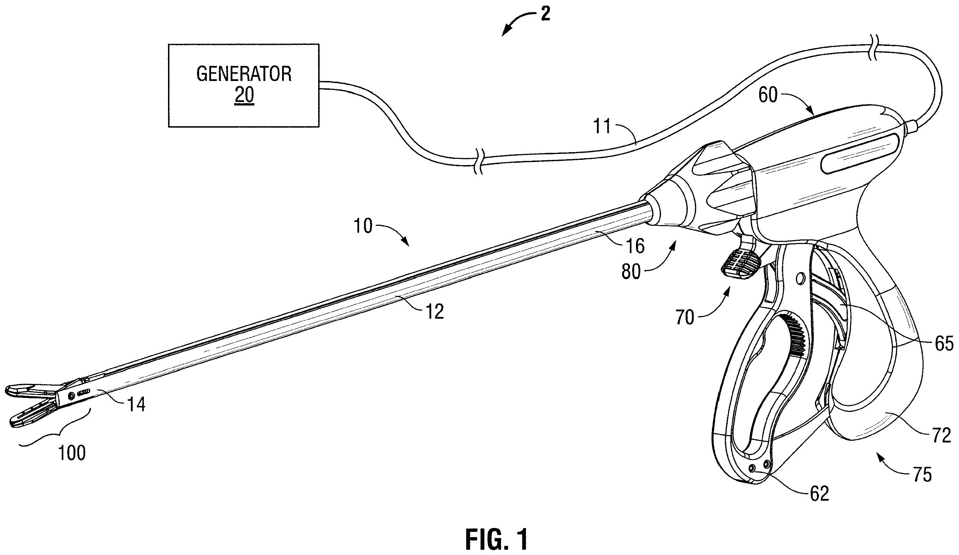

Referring now to Fig. 1, a tissue sealing system 2 according to the present disclosure is shown including a forceps 10 coupled to a generator 20. The forceps 10 is adapted to seal tissue using microwave energy. The generator 20 may be configured to output various types of microwave energy (e.g., from about 300 MHz to about 10,000 MHz).

The forceps 10 is coupled to the generator 20 via a cable 11 adapted to transmit energy and control signals therebetween. Various embodiments of the forceps 10 utilizing the aforementioned types of energy are discussed in more detail below.

The forceps 10 is configured to support an end effector assembly 100. Forceps 10 typically includes various conventional features (e.g., a housing 60, a handle assembly 75, a rotating assembly 80, a trigger assembly 70) that enable forceps 10 and end effector assembly 100 to mutually cooperate to grasp, seal and, if warranted, divide tissue. Forceps 10 generally includes housing 60 and handle assembly 75, which includes moveable handle 62 and handle 72 that is integral with housing 60. Handle 62 is moveable relative to handle 72 to actuate end effector assembly 100 to grasp and treat tissue. Forceps 10 also includes a shaft 12 that has distal end 14 that mechanically engages end effector assembly 100 and proximal end 16 that mechanically engages housing 60 proximate rotating assembly 80 disposed at the distal end of housing 60. Rotating assembly 80 is mechanically associated with shaft 12. Movement of rotating assembly 80 imparts similar rotational movement to shaft 12 which, in turn, rotates end effector assembly 100.

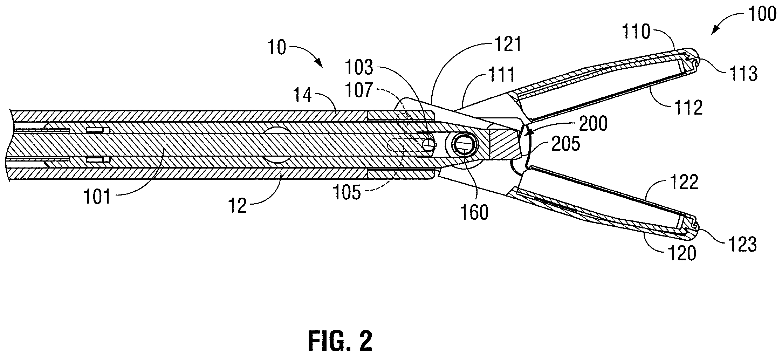

Referring to Fig. 2, the end effector assembly 100 includes two jaw members 110 and 120 having proximal ends 111, 121 and distal ends 113, 123. Jaw members 110 and 120 are pivotable about a post 160 and are movable from a first position wherein jaw members 110 and 120 are spaced relative to another, to a second position wherein jaw members 110 and 120 are closed and cooperate to grasp tissue therebetween. As discussed in more detail below, the end effector assembly 100 may be adapted for use with various energy sources.

The shaft 12 houses a pushrod 101 that is operatively coupled to the movable handle 62 such that when the handle 62 is moved relative to the handle 72 the pushrod 101 moves longitudinally, either proximally or distally within the shaft 12. The pushrod 101 includes a push pin 103 disposed at the distal end 16 of shaft 12. Each of the jaw members 110 and 120 includes a slot 105 and 107, respectively, disposed at the proximal ends thereof. The slots 105 and 107 are in mechanical cooperation with the push pin 103, which is adapted to move within the slots 105 and 107. The pin 103 and slots 105 and 107 operate as a cam-follower mechanical linkage. Motion of the pushrod 101 causes the pin 103 to slide within respective slots 105 and 107. The slots 105 and 107 may be angled with respect to the distal ends of the jaws members 110 and 120 such that the members 110 and 120 move either toward or away from each other as the pushrod 101 is moved longitudinally in a proximal or distal direction, respectively.

The forceps 10 also includes a trigger assembly 70 that advances a knife 200 disposed within the end effector assembly 100. Once a tissue seal is formed, the user activates the trigger assembly 70 to separate the tissue along the tissue seal. Knife 200 includes a sharpened edge 205 for severing the tissue held between the jaw members 110 and 120 at the tissue sealing site.

Each jaw member 110 and 120 includes a sealing surface 112 and 122, respectively, disposed on an inner-facing surface thereof. Sealing surfaces 112 and 122 cooperate to seal tissue held therebetween upon the application of energy. Sealing surfaces 112 and 122 are connected to generator 20 that communicates energy through the tissue held therebetween.

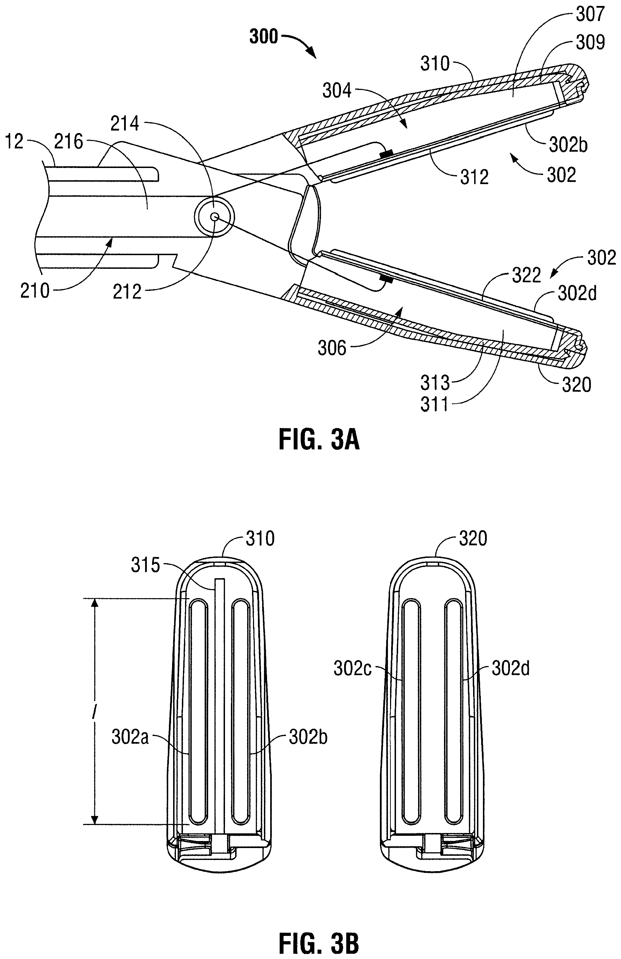

Figs. 3A and 3B illustrate a microwave end effector assembly 300 according to one embodiment of the present disclosure. The end effector assembly 300 is coupled to a coaxial cable 210 that is housed within the shaft 12 and the cable 11. The cable 210 includes an inner conductor 212 surrounded by an inner insulator 214, which is, in turn, surrounded by an outer conductor 216 (e.g., a cylindrical conducting sheath). The inner conductor 212 and outer conductor 216 may be constructed of copper, gold, stainless steel or other conductive metals with similar conductivity values. The metals may be plated with other materials, e.g., other conductive materials, to improve their properties, e.g., to improve conductivity or decrease energy loss, etc.

The end effector assembly 300 includes a microwave antenna assembly 302 having one or more microwave antennas 302a, 302b, 302c and 302d disposed on the sealing surfaces 312 and 322, respectively. In one embodiment, the microwave antennas 302a - 302d may have a length *l* of about ¼ of the wavelength of the microwave energy being supplied thereto. The microwave antennas 302a - 302d are coupled to the generator 20, which is adapted to supply microwave energy to the forceps 10 through the cable 210. The coaxial cable 210 connects one or more of the microwave antennas 302a - 302d to an active element of the generator 20 through the inner conductor 212 to form a first pole and the remaining microwave antennas 302a - 302d to a ground reference of the generator through the outer conductor 216 to form a second pole.

Fig. 3B shows a top view of the sealing surfaces 312 and 322 with the microwave antennas 302a - 302d configured as longitudinal strips that extend the lengths of the sealing surfaces 312 and 322. The microwave antennas 302a - 302d may be made from any type of conducting, non-reactive metals, such as stainless steel. The microwave antennas 302a - 302d may be configured either in a monopole or dipole arrangement. In a monopolar arrangement, a single microwave antenna, e.g., antenna 302a, is connected to the inner conductor 212 of the cable 210 and is disposed in a respective sealing surface 312.

In a dipole arrangement, two or more microwave antennas, e.g., antennas 302a and 302c may be used. One of the antennas may be the first pole (e.g., coupled to the inner conductor 212 of the cable 210) and another antenna may be a second pole (e.g., coupled to the outer conductor 216 of the cable 210). In one embodiment, the antenna 302a may be the first pole and the antenna 302c may be the second pole, such that the microwave energy flows from the sealing surface 312 to the sealing surface 322. When tissue is sealed by the assembly 300 in this dipole configuration, the antennas 302a and 302c may provide for an automatic termination of the sealing procedure. As sealing progresses, the tissue separating the antennas 302a and 302c is removed, thereby decreasing the separation between the antennas 302a and 302c. As the antennas 302a and 302c are moved toward each other by the compression forces, the microwave energy transmitted therethrough is reflected back therethrough and the radiation automatically stops due to the proximity of the first and second poles (e.g., antennas 302a and 302c).

In another embodiment, the antennas 302a and 302b may be configured as a planar dipole antenna such that the antennas 302a and 302b are disposed side-by-side on the sealing surface 312. More specifically, the antenna 302a may be the first pole and the antenna 302b may be the second pole, such that the energy flows across the sealing surface 112.

In another embodiment, multiple antennas may form the first and second pole, respectively. Any of the two antennas may form the first pole, with the remaining antennas forming the second pole. In particular, antennas 302a and 302b may form the first pole with antennas 302c and 302d forming the second pole, such that microwave energy flows between the sealing surfaces 312 and 322. In another embodiment, the first pole may include the antennas 302a and 302d, while the second pole includes antennas 302b and 302c. Those skilled in the art will appreciate that various other arrangements of antennas 302 are also possible.

The jaw members 310 and 320 also include shielding members 304 and 306 disposed therein, which include respective the sealing surfaces 312 and 322. Each of the shielding members 304 and 306 may include a dielectric portion 307 and 311 and a metallic plate 309 and 313 disposed over the dielectric portions 307 and 311, respectively. The dielectric portions 307 and 311 may be formed from a dielectric material that restricts propagation of microwave energy, such as ceramic. The shielding members 304 and 306, by nature of the relatively high dielectric properties and the presence of the metallic plate, reflect the microwave energy from the antennas 302a - 302d toward tissue being grasped between the sealing surfaces 312 and 322. This arrangement allows for use of any number of antennas 302 (e.g., a single antenna) since the microwave energy is restricted to the volume of tissue being grasped between the jaw members 310 and 320.

The end effector assembly 300 also includes a longitudinally-oriented channel 311 defined in the sealing surface 312 extending from the proximal end to the distal end thereof. The channel 315 facilitates longitudinal reciprocation of the knife 200 along a particular cutting plane to effectively and accurately separate the tissue along a formed tissue seal. The channel 315 may also be defined in the sealing surface 322 or solely disposed in only one sealing surface, e.g., sealing surface 312.

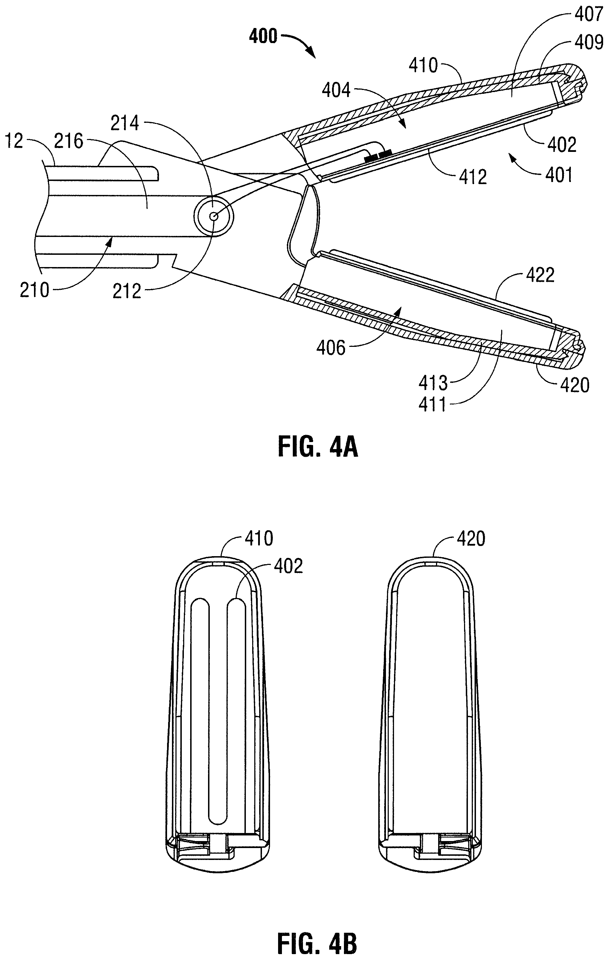

Figs. 4A and 4B illustrate a microwave end effector assembly 400 according to another embodiment of the present disclosure. The end effector assembly 400 includes jaw members 410 and 420 having shielding members 404 and 406 disposed therein, which include sealing surfaces 412 and 422, respectively. Each of the shielding members 404 and 406 may include a respective dielectric portion 407 and 411 and a metallic plate 409 and 413 disposed over the dielectric portions 407 and 411, respectively. The dielectric portions 407 and 411 may be formed from a dielectric material that restricts propagation of microwave energy, such as ceramic. The shielding members 404 and 406, by nature of the relatively high dielectric properties and the presence of the metallic plate, reflect the microwave energy toward tissue being grasped between the sealing surfaces 412 and 422.

The end effector assembly 400 is also coupled to the coaxial cable 210 and includes a microwave antenna assembly 401 having microwave antenna 402 disposed on the sealing surface 412. The microwave antenna 402 may be a so-called "microstrip" antenna, which is embedded in the sealing surface 422 of the shielding member 404. The antenna 402 is wound across the sealing surface 422 to maximize the surface area and the sealing area of the sealing surface 412. As shown in Fig. 4B, the antenna 402 may be wound longitudinally or transversely across the sealing surface 422. The antenna 402 may be a single pole antenna, in which case, the microwave energy is supplied thereto only though one of the conductors of the cable 210. The antenna 402 may be made from any type of conducting non-reactive metals, such as stainless steel.

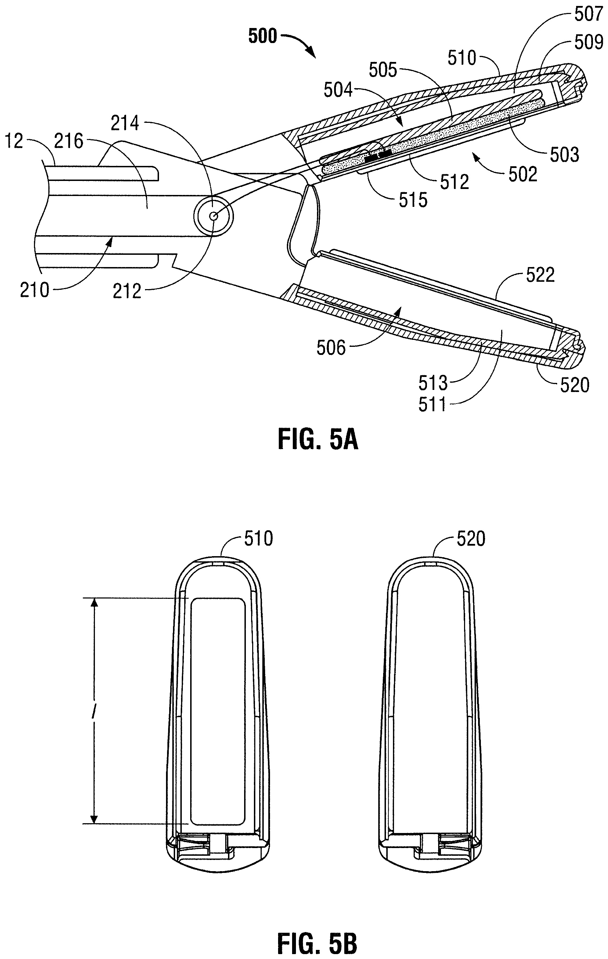

Figs. 5A and 5B illustrate a microwave end effector assembly 500 according to another embodiment of the present disclosure. The end effector assembly 500 includes jaw members 510 and 520 having shielding members 504 and 506 disposed therein, which include sealing surfaces 512 and 522, respectively. Each of the shielding members 504 and 506 may include a respective dielectric portion 507 and 511 and a metallic plate 509 and 513 disposed over the dielectric portions 507 and 511, respectively. The dielectric portions 507 and 511 may be formed from a dielectric material that restricts propagation of microwave energy, such as ceramic. The shielding members 504 and 506, by nature of the relatively high dielectric properties and the presence of the metallic plate, reflect the microwave energy toward tissue being grasped between the sealing surfaces 512 and 522.

The end effector assembly 500 is also coupled to the coaxial cable 210 and includes a microwave antenna assembly 502 disposed on the sealing surface 512. The microwave antenna assembly 502 includes a patch antenna 515 having a substantially rectangular shape. The microwave antenna assembly 502 also includes a dielectric substrate 503 and a grounding member 505. The patch antenna 515 is coupled to the inner conductor 212 of the cable 210 and the grounding member 505 is coupled to the outer conductor 214. The patch antenna 515 and the grounding member 505 are electrically insulated by the substrate 503. The substrate 503 may have a larger surface area than the patch antenna 515 such that the patch antenna 515 is completely covered by the substrate 503 to confine propagation of the microwave energy to the grounding member 505 to the substrate 503. In another embodiment, the substrate 503 and the grounding member 505 may be replaced by the shielding member 504. In other words, the grounding member 505 may be enclosed within the shielding member 504 and the patch antenna 515 may then be disposed on top thereof. The patch antenna 515 may be made from any type of conducting non-reactive metals, such as stainless steel. The grounding member 505 may be may be constructed of copper, gold, stainless steel or other conductive metals with similar conductivity values. The metals may be plated with other materials, e.g., other conductive materials, to improve their properties, e.g., to improve conductivity or decrease energy loss, etc.

The patch antenna 515 may have a length *l* that is substantially equal to ½ of the wavelength of the microwave energy being supplied thereto. The wavelength also depends on the dielectric properties of the substrate 503 and/or the shielding member 504. The relationship between the wavelength and the dielectric properties of the materials is expressed by the formula (1):

$$(1) \; \lambda_s = c \, / \, (f \, \sqrt{\varepsilon_s})$$

wherein c is a constant representing the speed of light, f is the frequency of the microwave energy, and $\varepsilon_s$ is a dielectric permittivity of the substrate 503 and/or the shielding member 504. The formula (1) illustrates that the wavelength $\lambda_s$ may be varied by selecting different frequencies, f, and/or dielectric materials $\varepsilon_s$.

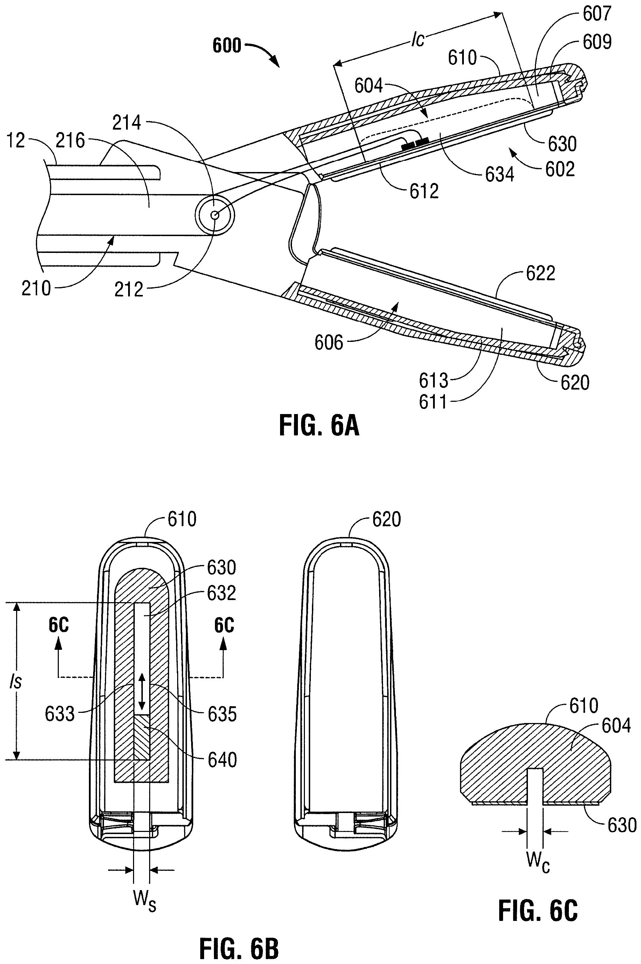

Figs. 6A - 6C illustrate a microwave end effector assembly 600 according to yet another embodiment the present disclosure. The end effector assembly 600 includes jaw members 610 and 620 having shielding members 604 and 606 disposed therein, which include sealing surfaces 612 and 622, respectively. Each of the shielding members 604 and 606 may include a respective dielectric portion 607 and 611 and a metallic plate 609 and 613 disposed over the dielectric portions 607 and 611, respectively. The dielectric portions 607 and 611 may be formed from a dielectric material that restricts propagation of microwave energy, such as ceramic. The shielding members 604 and 606, by nature of the relatively high dielectric properties and the presence of the metallic plate, reflect the microwave energy toward tissue being grasped between the sealing surfaces 612 and 622.

The end effector assembly 600 is also coupled to the coaxial cable 210 and includes a microwave antenna assembly 602 disposed on the sealing surface 612. The microwave antenna assembly 602 includes a slot antenna 630 having a substantially rectangular slot 632 therein as shown in Fig. 6B. The slot antenna 630 may be made from any type of conducting non-reactive metals, such as stainless steel. The rectangular slot 632 has a length $l_s$ and a width $w_s$.

The microwave antenna assembly 602 may also include a cavity 634 formed within the shielding member 604 of the jaw member 610. In one embodiment, the cavity 634 may extend in a proximal direction to facilitates longitudinal reciprocation of the knife 200 along a particular cutting plane to effectively and accurately separate the tissue along a formed tissue seal. The length $l_c$ and width $w_c$ of the cavity 634 (Figs. 6A and 6C) are substantially equal to the length $l_s$ and the width $w_s$ of the slot 632 (Fig. 6B), such that the slot 632 substantially overlaps the cavity 634. The rectangular slot 632 also includes a first and second longitudinal sides 633 and 635. The first side 633 is coupled to the inner conductor 212 of the cable 210 and the second side 635 is coupled to the outer conductor 214, such that the first side 633 acts as a first pole and the second side 635 acts as a second pole. As microwave energy is supplied to the slot antenna 630, the microwave energy is transmitted from the first side 633 across to the second side 635 and into the cavity 634. In addition, the overlapping of the slot 632 and the cavity 634 allows for directional radiation of microwave energy from the slot antenna 630 toward the jaw member 620 as the microwave energy is bounced downward by the cavity 634. The cavity 634 allows for concentration of the microwave energy down the center of the jaw members 610 and 620, providing for a narrower seal.

The length $l_c$ of the cavity 634 and the length $l_s$ of the slot 632 is substantially equal to ½ of the wavelength of the microwave energy being supplied thereto. The wavelength also depends on the dielectric properties of the surrounding environment and/or the shielding member 604. As microwave energy is applied to the tissue, the tissue is desiccated, which, in turn, changes the dielectric properties of the surrounding environment. Therefore, as illustrated by formula (1) above, based on the relationship between the dielectric permittivity of the surrounding environment, the wavelength of the microwave energy being supplied to the tissue is also affected. Accordingly, to maintain the match between the length $l_c$ of the cavity 634 and the length $l_s$ of the slot 632 and ½ of the wavelength of the microwave energy, the length $l_c$ of the cavity 634 and the length $l_s$ of the slot 632 may be adjusted during operation.

As best shown in Fig. 6B, the antenna assembly 602 includes a retractable plate 640 housed between the shielding member 604 and the slot antenna 630. The retractable plate 640 is movable in a longitudinal direction between the shielding member 604 and the slot antenna 630 such that the opening defined by the slot 632 into the cavity 634 is at least partially covered up. The retractable plate 640 has a width larger than the width $w_s$ of the slot 632, such that when the retractable plate 640 is slid between the shielding member 604 and the slot antenna 630, the retractable plate 640 fully covers the slot 632 up to the point of extension of the retractable plate 640. The length of the retractable plate 640 may be any suitable length, such as the length $l_s$ of the slot 632. This allows for the retractable plate 640 to fully cover the slot 632 when being fully retracted.

During operation, the retraction of the retractable plate 640 may be adjusted to match the antenna assembly 602 to the wavelength of the microwave energy as the dielectric properties of the surrounding media is changing. More specifically, adjusting the length of retraction of the retractable plate 640 adjusts the length $l_c$ of the cavity 634 and the length $l_s$ of the slot 632 to maintain theses lengths substantially equal to ½ of the wavelength of the microwave energy, as the wavelength of the microwave energy is changing due to the changes in the dielectric properties.

The above embodiments of the microwave antenna assemblies may also be utilized to measure certain tissue properties such as temperature and dielectric properties. The microwave antenna assembly is configured to operate in a therapeutic mode to deliver microwave energy to seal tissue and in a detection mode to measure tissue properties.

In one embodiment, the microwave antenna assemblies may be utilized in a receiving mode only. In other words, the antenna assembly may be configured as a radiometer to detect changes in electromagnetic radiation emanating from the tissue. The detected changes in electromagnetic radiation are then processed by the generator 20 to calculate the temperature of the tissue.

In another embodiment, the microwave antenna assemblies may be configured to detect dielectric properties of the tissue. This may be accomplished by transmitting non-therapeutic microwave energy into the tissue and then measuring reflected and forward power. The reflected and forward power is indicative of the dielectric properties of tissue and may be measured based on the impedance mismatch between the generator 20 and tissue due to the changes in dielectric properties of the tissue and other system components. More specifically, impedance mismatches cause a portion of the power, so-called "reflected power," from the generator 20 to not reach the load and cause the power or energy delivered, the so-called "forward power," to vary in an irregular or inconsistent manner over the treatment time interval. The actual power of the generator may be expressed as a sum of the forward power and reflected power. Thus, it is possible to determine the impedance mismatch that is caused by dielectric properties of the tissue by measuring and analyzing the reflected and forward power. This may be accomplished by transmitting a non-therapeutic microwave pulse (e.g., 1 GHz - 5 GHz) and then measuring the reflected and forward power at the generator 20. The generator 20 then accounts for the impedance mismatch caused by system components (e.g., cable and antenna assembly stray capacitance) to calculate the portion of the mismatch due to the dielectric properties of the tissue. This then allows the generator 20 to determine those properties.

While several embodiments of the disclosure have been shown in the drawings and/or discussed herein, it is not intended that the disclosure be limited thereto. The above description should not be construed as limiting, but merely as exemplifications of particular embodiments.

**Claims**

1. A microwave forceps for sealing tissue, comprising:

    at least one shaft member having an end effector assembly (500) disposed at a distal end thereof, the end effector assembly (500) including first and second opposing jaw members (510,520) movable from a first position in spaced relation relative to one another to at least one subsequent position wherein the jaw members cooperate to grasp tissue therebetween, each of the jaw members including a sealing surface (512,522); and

a microwave antenna assembly (502) configured to couple to a microwave energy source, the microwave antenna assembly disposed on the sealing surface (512) of the first jaw member (510), **characterized in that** said microwave antenna assembly (502) comprises a patch antenna (515) coupled to an active element of the microwave energy source.

2. The microwave forceps according to claim 1, wherein the microwave antenna assembly (502) includes a grounding member (505) operably coupled between the first jaw member (510) and a ground reference of the microwave energy source.

3. The microwave forceps according to claim 2, wherein the microwave antenna assembly (502) includes a dielectric substrate (503) disposed between the grounding member (505) and the patch antenna (515).

4. The microwave forceps according to claim 3, wherein the patch antenna (515) is disposed on the dielectric substrate (503).

5. The microwave forceps according to claim 4, wherein the dielectric substrate (503) has a larger surface area than a surface area of the patch antenna (515).

6. The microwave forceps according to claim 1, wherein the microwave antenna assembly (502) includes a shielding member (504), wherein the patch antenna (515) is disposed on top of the shielding member (504).

7. The microwave forceps according to any one of claims 1 to 6, wherein the patch antenna (515) has a length that is substantially equal to about half of a wavelength of the microwave energy supplied thereto.

8. The microwave forceps according to claim 2, 3, 4 or 5, wherein the end effector assembly (500) is also coupled to a coaxial cable (210) and the patch antenna (515) is coupled to an inner conductor (212) of the coaxial cable (210) and the grounding member (505) is coupled to an outer conductor (214) of the coaxial cable.

9. The microwave forceps according to any one of claims 2, 4, 5 and 8, wherein the patch antenna (515) and the grounding member (505) are electrically insulated by the dielectric substrate (503).

10. The microwave forceps according to any one of the preceding claims, wherein the patch antenna has a rectangular shape.

11. The microwave forceps according to any preceding claim,

wherein the jaw members (510, 520) have shielding members (504, 506) disposed therein, which include the sealing surfaces (512, 522), respectively,

wherein each of the shielding members (504, 506) include a respective dielectric portion (507,511) and a metallic plate (509, 513) disposed over the dielectric portions (507, 511), respectively, whereby the shielding members (504, 506), by nature of relatively high dielectric properties and the presence of the metallic plate, reflect the microwave energy toward tissue being grasped between the sealing surfaces (512, 522).


**Patentansprüche**

1. Mikrowellenzange zum Versiegeln von Gewebe, mit:

mindestens einem Schaftelement, das an seinem distalen Ende einen Endeffektoraufbau (500) angeordnet aufweist, wobei der Endeffektoraufbau (500) sich gegenüberstehende Backenelemente (510, 520) aufweist, die von einer ersten relativ zueinander beabstandeten Beziehung zu mindestens einer nachfolgenden Position bewegbar sind, wobei die Backenelemente zusammenwirken, um dazwischen Gewebe zu greifen, wobei jedes der Backenelemente eine Versiegelungsfläche (512, 522) aufweist; und einen Mikrowellenantennenaufbau (502), der konfiguriert ist, an eine Mikrowellenenergiequelle zu koppeln, wobei der Mikrowellenantennenaufbau auf der Versiegelungsfläche (512) des ersten Backenelements (510) angeordnet ist, **dadurch gekennzeichnet, dass** der Mikrowellenantennenaufbau (502) eine Patch-Antenne (515) umfasst, die mit einem aktiven Element der Mikrowellenenergiequelle gekoppelt ist.

2. Mikrowellenzange nach Anspruch 1, wobei der Mikrowellenantennenaufbau (502) ein Erdungselement (505) umfasst, das betriebsmäßig zwischen dem ersten Backenelement (510) und einer Erdungsreferenz der Mikrowellenenergiequelle gekoppelt ist.

3. Mikrowellenzange nach Anspruch 2, wobei der Mikrowellenantennenaufbau (502) ein dielektrisches Substrat (503) umfasst, das zwischen dem Erdungselement (505) und der Patch-Antenne (515) angeordnet ist.

4. Mikrowellenzange nach Anspruch 3, wobei die Patch-Antenne (515) an dem dielektrischen Substrat (503) angeordnet ist.

5. Mikrowellenzange nach Anspruch 4, wobei das dielektrische Substrat (503) einen größeren Oberflächenbereich als einen Oberflächenbereich der Patch-Antenne (515) aufweist.

6. Mikrowellenzange nach Anspruch 1, wobei der Mikrowellenantennenaufbau (502) ein Abschirmelement (504) aufweist, wobei die Patch-Antenne (515) oben auf dem Abschirmelement (504) angeordnet ist.

7. Mikrowellenzange nach einem der Ansprüche 1 bis 6, wobei die Patch-Antenne (515) eine Länge aufweist, die im Wesentlichen gleich zu ungefähr der Hälfte einer Wellenlänge der Mikrowellenenergie ist, die dorthin geführt wird.

8. Mikrowellenzange nach Anspruch 2, 3, 4 oder 5, wobei der Endeffektoraufbau (500) ebenfalls an ein Koaxialkabel (210) gekoppelt ist und die Patch-Antenne (515) mit einem Innenleiter (212) des Koaxialkabels (210) gekoppelt ist und das Erdungselement (505) mit einem Außenleiter (214) des Koaxialkabels gekoppelt ist.

9. Mikrowellenzange nach einem der Ansprüche 2, 4, 5 und 8, wobei die Patch-Antenne (515) und das Erdungselement (505) durch das dielektrische Substrat (503) elektrisch isoliert sind.

10. Mikrowellenzange nach einem der vorhergehenden Ansprüche, wobei die Patch-Antenne eine rechteckige Form aufweist.

11. Mikrowellenzange nach einem der vorhergehenden Ansprüche,
wobei die Backenelemente (510, 520) Abschirmelemente (504, 506) darin angeordnet aufweisen, welche die entsprechenden Versiegelungsflächen (512, 522) umfassen,
wobei jedes der Abschirmelemente (504, 506) einen entsprechenden dielektrischen Abschnitt (507, 511) und eine Metallplatte (509, 513) aufweist, die über den jeweiligen dielektrischen Abschnitten (507, 511) angeordnet sind, wodurch die Abschirmelemente (504, 506), die aufgrund ihrer Beschaffenheit relativ hohe dielektrische Eigenschaften besitzen und dem Vorhandensein der Metallplatte, die Mikrowellenenergie in Richtung des Gewebes reflektieren, das zwischen den Versiegelungsflächen (512, 522) ergriffen wurde.

**Revendications**

1. Forceps à micro-ondes pour le scellage d'un tissu, comprenant :

au moins un élément d'arbre ayant un ensemble effecteur terminal (500) disposé à son extrémité distale, l'ensemble effecteur terminal (500) comprenant un premier et un second élément de mors opposés (510,520) mobiles d'une première position espacée l'une de l'autre à au moins une autre position, dans lequel les éléments de mors coopèrent pour saisir un tissu disposé entre eux, chacun des éléments de mors comprenant une surface de scellage (512, 522) ; et
un ensemble d'antenne à micro-ondes (502) configuré pour se coupler à une source d'énergie à micro-ondes, l'ensemble d'antenne à micro-ondes étant disposé sur la surface de scellage (512) du premier élément de mors (510), **caractérisé en ce que** ledit ensemble d'antenne à micro-ondes (502) comprend une antenne en plaque (515) couplée à un élément actif de la source d'énergie à micro-ondes.

2. Forceps à micro-ondes selon la revendication 1, dans lequel l'ensemble d'antenne à micro-ondes (502) comprend un élément de mise à la terre (505) couplé en service entre le premier élément de mors (510) et une référence de terre de la source d'énergie à micro-ondes.

3. Forceps à micro-ondes selon la revendication 2, dans lequel l'ensemble d'antenne à micro-ondes (502) comprend un substrat diélectrique (503) disposé entre l'élément de mise à la terre (505) et l'antenne en plaque (515).

4. Forceps à micro-ondes selon la revendication 3, dans lequel l'antenne en plaque (515) est disposée sur le substrat diélectrique (503).

5. Forceps à micro-ondes selon la revendication 4, dans lequel le substrat diélectrique (503) a une surface plus grande qu'une surface de l'antenne en plaque (515).

6. Forceps à micro-ondes selon la revendication 1, dans lequel l'ensemble d'antenne à micro-ondes (502) comprend un élément de protection (504), dans lequel l'antenne en plaque (515) est disposée par-dessus l'élément de protection (504).

7. Forceps à micro-ondes selon l'une quelconque des revendications 1 à 6, dans lequel l'antenne en plaque (515) a une longueur qui est sensiblement égale à environ la moitié d'une longueur d'onde de l'énergie à micro-ondes qui lui est fournie.

8. Forceps à micro-ondes selon la revendication 2, 3, 4 ou 5, dans lequel l'ensemble effecteur d'extrémité (500) est également couplé à un câble coaxial (210) et l'antenne en plaque (515) est couplée à un conducteur interne (212) du câble coaxial (210) et l'élément de mise à la terre (505) est couplé à un conducteur externe (214) du câble coaxial.

9. Forceps à micro-ondes selon l'une quelconque des revendications 2, 4, 5 ou 8, dans lequel l'antenne en plaque (515) et l'élément de mise à la terre (505) sont isolés électriquement par le substrat diélectrique (503).

10. Forceps à micro-ondes selon l'une quelconque des revendications précédentes, dans lequel l'antenne en plaque a une forme rectangulaire.

11. Forceps à micro-ondes selon l'une quelconque des revendications précédentes, dans lequel les éléments de mors (510, 520) ont des éléments de protection (504, 506) qui y sont disposés et qui comprennent les surfaces de scellage (512, 522), respectivement,
dans lequel chacun des éléments de protection (504, 506) comprend une partie diélectrique respective (507, 511) et une plaque métallique (509, 513) disposée par-dessus les parties diélectriques (507, 511), respectivement, en sorte que les éléments de protection (504, 506), du fait des propriétés diélectriques relativement élevées et de la présence de la plaque métallique, réfléchissent l'énergie à micro-ondes vers le tissu qui est saisi entre les surfaces de scellage (512, 522).

**FIG. 1**

EP 2 868 287 B1

**FIG. 2**

EP 2 868 287 B1

**FIG. 3A**

**FIG. 3B**

**FIG. 4A**

**FIG. 4B**

**500**

FIG. 5A

FIG. 5B

**FIG. 6A**

**FIG. 6B**

**FIG. 6C**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070225697 A
- WO 2008044000 A
- JP 2008054926 A